# EUROPEAN PATENT APPLICATION

(11) **EP 4 799 595 A1**
(43) Date of publication of application: **02.09.2026**
(21) Application number: 25199921.5
(22) Date of filing: 03.09.2025
(51) Int. Cl.: A61F 9/02, G02C 7/02

(54) **GOGGLE LENS**

(30) Priority: 26.02.2025 TW 114201978 U
(71) Applicant: CHIU YU, Hui-Hsuan, Tainan City 708013 (TW)
(72) Inventor: CHIU YU, Hui-Hsuan, Tainan City 708013 (TW)
(74) Representative: Stöckeler, Ferdinand

(57) **Abstract**

A goggle lens coupled with a goggle frame (8) for enclosing a wearer's head includes a central lens portion (1) and two lateral lens portions (2) respectively extending from left and right sides of the central lens portion (1). The central lens portion (1) includes a central convex surface (12) distal to the wearer's face and having a first horizontal radius of curvature. Each lateral lens portion (2) includes a first lateral lens section (21) which has a first convex surface (212) distal to the wearer's face and has a second horizontal radius of curvature. The second horizontal radius of curvature is smaller than the first horizontal radius of curvature such that the central lens portion (1) is closer and more fittingly attached to the wearer's face to improve the aesthetic appearance of the goggle lens.

## Description

The disclosure relates to a goggle lens, and more particularly to a goggle lens coupled with a goggle frame for enclosing a wearer's head.

A conventional goggle lens is coupled with a goggle frame for enclosing a wearer's head. The conventional goggle lens generally has a convex surface with a constant transverse curvature such that the central area of the convex surface appears relatively far from the wearer's face and seems protruding, so the conventional goggle lens is not fittingly attached to the wearer's face. Therefore, there is room for improvement for the conventional goggle lens.

Therefore, an object of the disclosure is to provide a goggle lens that can alleviate at least one of the drawbacks of the prior art.

According to the disclosure, the goggle lens goggle lens is adapted to be coupled with a goggle frame for enclosing a wearer's head, and includes a central lens portion and two lateral lens portions. The central lens portion includes a central convex surface distal to the wearer's face. The central convex surface has a first horizontal radius of curvature which is measured by a curvature of a horizontal normal section between a transverse normal plane and any point on the central convex surface. The lateral lens portions respectively extend from a left side and a right side of the central lens portion. Each of the lateral lens portions includes a first lateral lens section which has a first convex surface distal to the wearer's face. The first convex surface has a second horizontal radius of curvature which is measured by a curvature of a horizontal normal section between a transverse normal plane and any point on the first convex surface. The second horizontal radius of curvature is smaller than the first horizontal radius of curvature.

The central lens portion is closer and more fittingly attached to the wearer's face to reduce the visual deviation of the optical imaging and improve the outer appearance of wearing.

Other features and advantages of the disclosure will become apparent in the following detailed description of the embodiments with reference to the accompanying drawings. It is noted that various features may not be drawn to scale.
FIG. 1 is a perspective view illustrating a goggle with an embodiment of a goggle lens according to the disclosure enclosing a wearer's head.
FIG. 2 is a perspective view of the embodiment.
FIG. 3 is a front view of the embodiment.
FIG. 4 is a sectional view taken along line IV-IV of FIG. 3.
FIG. 5 is a sectional view taken along line V-V of FIG. 3.
FIG. 6 is a fragmentary sectional view illustrating a central convex surface, one first convex surface and one second convex surface of the embodiment.
FIG. 7 is a sectional view illustrating the central convex surface of the embodiment.
FIG. 8 is a perspective view illustrating another embodiment of a goggle lens according to the disclosure.
FIG. 9 is a front view of FIG. 8.
FIG. 10 is a sectional view taken along line X-X of FIG. 9.

Before the disclosure is described in greater detail, it should be noted that where considered appropriate, reference numerals or terminal portions of reference numerals have been repeated among the figures to indicate corresponding or analogous elements, which may optionally have similar characteristics.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

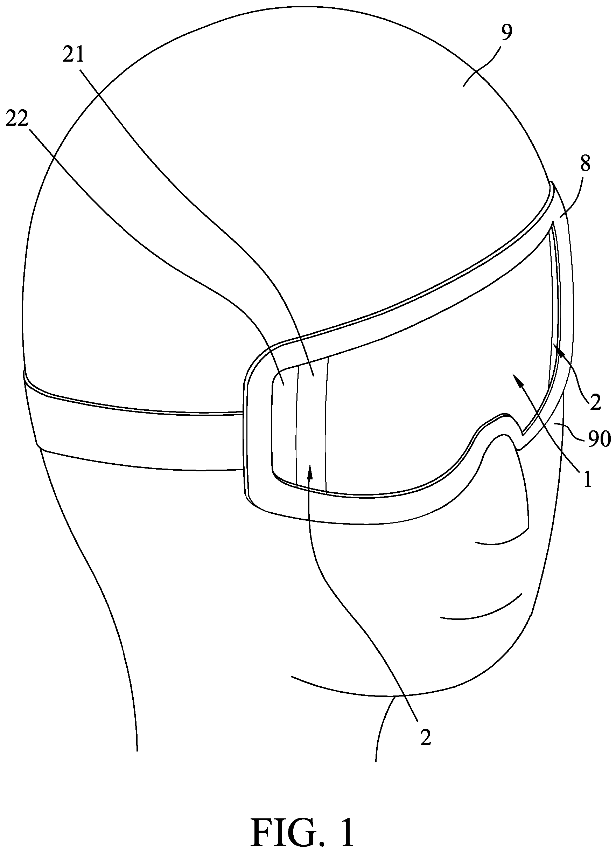

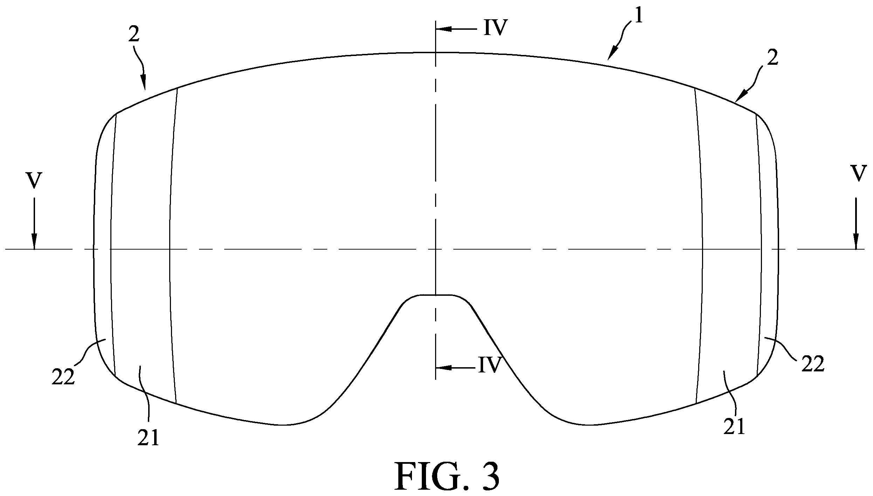

Referring to FIGS. 1 to 3, an embodiment of a goggle lens according to the disclosure is adapted to be coupled with a goggle frame 8 for enclosing a wearer's head 9. The goggle lens includes a central lens portion 1 and two lateral lens portions 2.

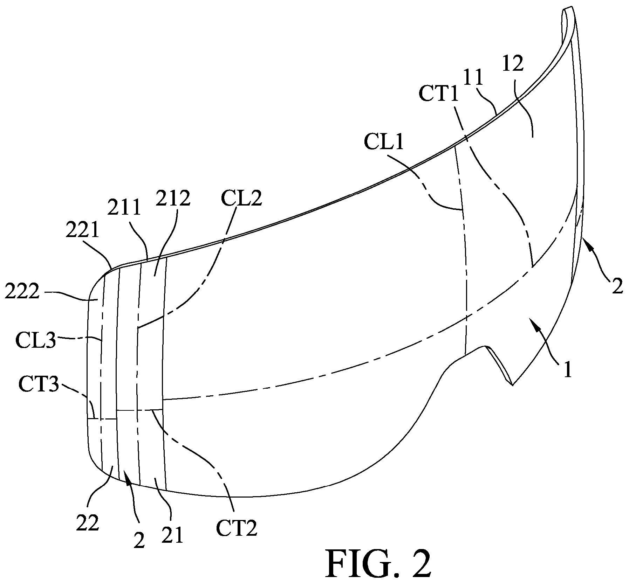

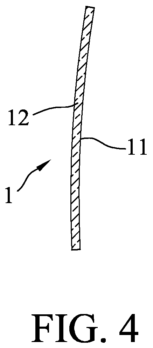

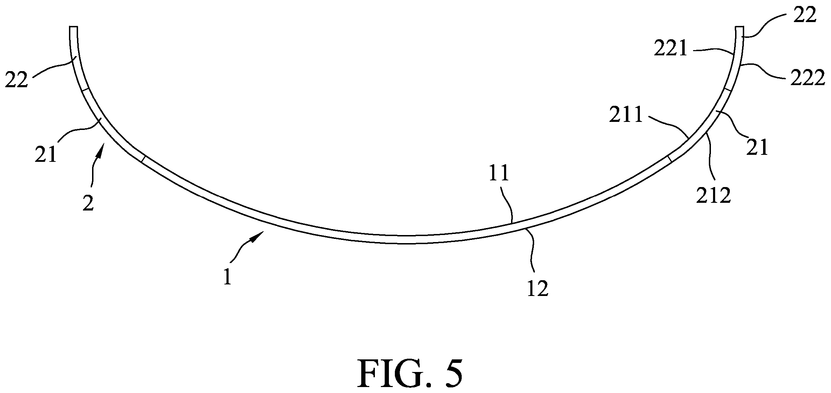

With reference to FIGS. 2, 4 and 5, the central lens portion 1 includes a central concave surface 11 that faces the wearer's face 90, and a central convex surface 12 that is distal to the wearer's face 90 and opposite to the central concave surface 11. The central convex surface 12 has a first horizontal radius of curvature and a first vertical radius of curvature. The first vertical radius of curvature is not smaller than the first horizontal radius of curvature.

Specifically, the first horizontal radius of curvature is defined by a curvature of a horizontal normal section between a transverse normal plane and any point on the central convex surface 12. In the embodiment, the horizontal normal section is a first transverse curved line (CT1). Thus, the first horizontal radius of curvature of any point on the first transverse curved line (CT1) is substantially constant.

The first vertical radius of curvature is defined by a curvature of a vertical normal section between a longitudinal normal plane and any point on the central convex surface 12. In the embodiment, the vertical normal section is a first longitudinal curved line (CL1). Thus, the first vertical radius of curvature of any point on the first longitudinal curved line (CL1) is substantially constant.

The two lateral lens portions 2 respectively extend from left and right sides of the central lens portion 1. Each lateral lens portion 2 includes a first lateral lens section 21 and a second lateral lens section 22 which extends laterally from the first lateral lens section 21 and is distal from the central lens portion 1.

Each first lateral lens section 21 has a first concave surface 211 that faces the wearer's face 90, and a first convex surface 212 that is distal to the wearer's face 90 and opposite to the first concave surface 211. The first convex surface 212 has a second horizontal radius of curvature and a second vertical radius of curvature. The second horizontal radius of curvature is smaller than the first horizontal radius of curvature. The second vertical radius of curvature is equal to the first vertical radius of curvature. Hence, the central convex surface 12 of the central lens portion 1 is closer to the wearer's face 90 to improve the aesthetic appearance of the goggle lens.

Specifically, in each first lateral lens section 21, the second horizontal radius of curvature is defined by a curvature of a horizontal normal section between a transverse normal plane and any point on the first convex surface 212. In the embodiment, the horizontal normal section is a second transverse curved line (CT2). Thus, the first horizontal radius of curvature of any point on the second transverse curved line (CT2) is substantially constant. In the embodiment, the first horizontal radius of curvature of any point on the first convex surface 212 is constant.

In each first lateral lens section 21, the second vertical radius of curvature is defined by a curvature of a vertical normal section between a longitudinal normal plane and any point on the first convex surface 212. In the embodiment, the vertical normal section is a second longitudinal curved line (CL2). Thus, the second vertical radius of curvature of any point on the second longitudinal curved line (CL2) is substantially constant. In the embodiment, the second vertical radius of curvature of any point on the first convex surface 212 is substantially constant.

Each second lateral lens section 22 has a second concave surface 221 that faces the wearer's face 90, and a second convex surface 222 that is distal to the wearer's face 90 and opposite to the second concave surface 221. The second convex surface 222 has a third horizontal radius of curvature and a third vertical radius of curvature.

Specifically, in each second lateral lens section 22, the third horizontal radius of curvature is defined by a curvature of a horizontal normal section between a transverse normal plane and any point on the second convex surface 222. In the embodiment, the horizontal normal section is a third transverse curved line (CT3). In some embodiments, the radius of curvature of the third transverse curved line (CT3) on the second convex surface 222 may gradually vary as requirement of use.

In each second lateral lens section 22, the third vertical radius of curvature is defined by a curvature of a vertical normal section between a longitudinal normal plane and any point on the second convex surface 222. In the embodiment, the vertical normal section is a third longitudinal curved line (CL3). Thus, the third vertical radius of curvature of any point on the third longitudinal curved line (CL3) is substantially constant. In the embodiment, the third vertical radius of curvature of any point on the second convex surface 222 is substantially constant.

The third horizontal radius of curvature of each second lateral lens section 22 is smaller than the first horizontal radius of curvature of the central convex surface 12. The third vertical radius of curvature of each second lateral lens section 22 is equal to the first vertical radius of curvature of the central convex surface 12.

Specifically, the first horizontal radius of curvature of the central convex surface 12 ranges from 109 mm to 234 mm. The first vertical radius of curvature of the central convex surface 12 ranges from 163 mm to 586 mm. The second horizontal radius of curvature of the first convex surface 212 ranges from 41 mm to 73 mm. The third horizontal radius of curvature of the second convex surface 222 ranges from 41 mm to 73 mm, and is different from the second horizontal radius of curvature of the first convex surface 212.

Furthermore, the refractive index of the material of the goggle lens may also affect usage of the goggle lens, and the above-mentioned radius of curvature is defined by a degree of curve. The formula for calculating the degree of curve of a convex surface is C = (n - 1) *1000/R (mm -1), where n is the refractive index of the goggle lens, and R is the radius of curvature of the corresponding curved line. The degree of curve of the first transverse curved line (CT1) ranges from 2.5 to 4.5, the degree of curve of each second transverse curved line (CT2) ranges from 8 to 12, and the degree of curve of each third transverse curved line (CT3) ranges from 8 to 12. The degree of curve of each of the first longitudinal curved line (CL1), the second longitudinal curved lines (CL2) and the third longitudinal curved lines (CL3) ranges from 1 to 3.

The goggle lens is made from polycarbonate, glass or poly(methyl methacrylate) (PMMA) material. In some embodiments, the material of the goggle lens includes additives. In the embodiment, the goggle lens is made from polycarbonate material. The goggle lens has a refractive index ranging from 1.4 to 1.6.

In some embodiments, the goggle lens is made from polycarbonate material having a refractive index of 1.586. The first horizontal radius of curvature of the central convex surface 12 ranges from 130 mm to 234 mm. The first vertical radius of curvature of the central convex surface 12 ranges from 195 mm to 586 mm. The second horizontal radius of curvature of the first convex surface 212 of each lateral lens portion 2 ranges from 49 mm to 73 mm. The third horizontal radius of curvature of the second convex surface 222 of each lateral lens portion 2 ranges from 49 mm to 73 mm. Hence, with the first horizontal radius of curvature and the first vertical radius of curvature of the central convex surface 12, the wearer is provided with a good visual image experience when the wearer 9 looks straight forward. With the second horizontal radius of curvature of the first convex surfaces 212 and the third horizontal radius of curvature of the second convex surfaces 222, visual deviation is eliminated when the wearer 9 looks to the left and the right.

In the embodiment, the first horizontal radius of curvature of the central convex surface 12 is 167 mm. The first vertical radius of curvature of the central convex surface 12 is 234 mm. The second horizontal radius of curvature of the first convex surface 212 is 65 mm. The third horizontal radius of curvature of the second convex surface 222 is 53 mm.

In some embodiments, the goggle lens is made from an acrylic material with a refractive index of 1.49, or a glass material with a refractive index of 1.52.

With reference to FIGS. 2, 6 and 7, specifically, each of the central convex surface 12, the first convex surfaces 212 and the second convex surfaces 222 is a double curvature surface.

In the embodiment, the first transverse curved line (CT1) is a part of a first imaging circle (O1). The radius of the first imaging circle (O1) is 167 mm. The second transverse curved line (CT2) is a part of a second imaging circle (O2). The radius of the second imaging circle (O2) is 65 mm. The third transverse curved line (CT3) is a part of a third imaging circle (O3). The radius of the third imaging circle (O3) is 53 mm. The first longitudinal curved line (CL1), each second longitudinal curved line (CL2) and each third longitudinal curved line (CL3) are parts of a fourth imaging circle (O4). The radius of the fourth imaging circle (O4) is 234 mm.

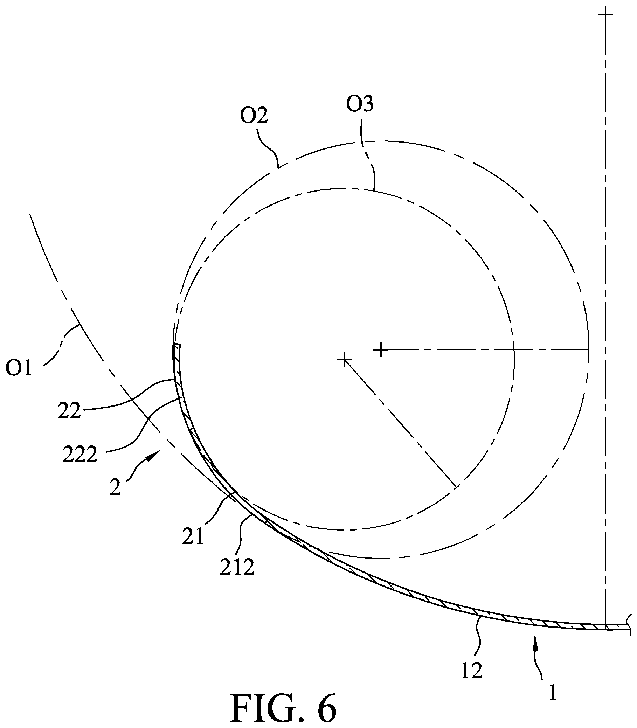

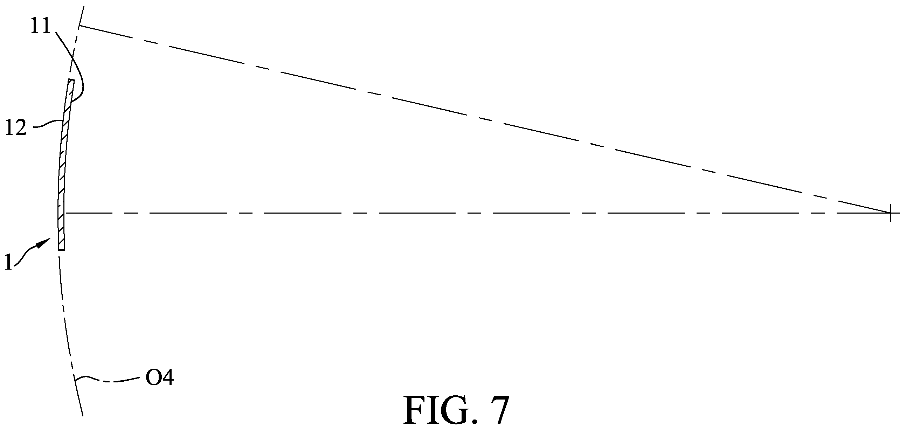

Since the goggle lens is of a left-right symmetric configuration, FIG. 6 merely illustrates a left half of the goggle lens. Since the radius of curvature of the first longitudinal curved line (CL1) is equal to the radius of curvature of each second longitudinal curved line (CL2) and each third longitudinal curved line (CL3), FIG. 7 merely illustrates the central lens portion 1.

The goggle lens is relatively thin, and hence it is understood that only the radius of curvature of the convex surfaces of the central lens portion 1 and the lateral lens portions 2 are taken into consideration, and the radius of curvature of each of the concave surfaces 11, 211, 221 of the central lens portion 1 and the lateral lens portions 2 are not taken into consideration.

The goggle lens is coupled with the goggle frame 8 by snap-fitting, magnet attraction or screw engagement for enclosing a wearer's head 9 to protect his/her eyes in sports, driving or working. With the central lens portion 1 and the lateral lens portions 2 of different radius of curvatures, the goggle lens is more fittingly attached to the wearer's face so as to improve the optical image. In the known art, with a goggle lens having a single horizontal radius of curvature, reducing the radius of curvature reduces the field of view on the left and right sides of the goggle lens. Alternatively, the thickness of the goggle frame 8 needs to be increased. In this embodiment, with the lateral lens portions 2 having the radius of curvature different from that of the central lens portion 1, the lateral lens portions 2 may be made with a greater radius of curvature and be curved inwardly such that the field of views on the left and right sides of the goggle lens are not reduced while the goggle lens is coupled with the goggle frame 8 having the same size.

In the embodiment, the central lens portion 1 is integrally formed and connected with each lateral lens portion 2, and a smooth curved surface is formed between the central lens portion 1 and each lateral lens portion 2 so as to prevent visual obstruction at the junction.

In some embodiments, the second lateral lens sections 22 are dispensed with.

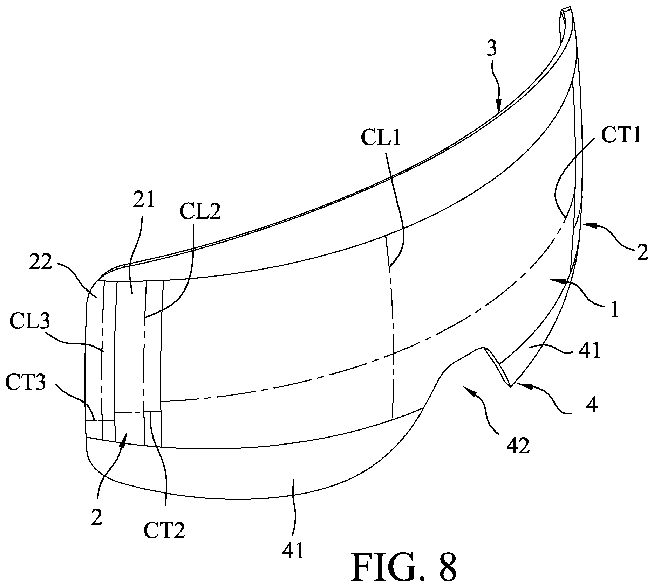

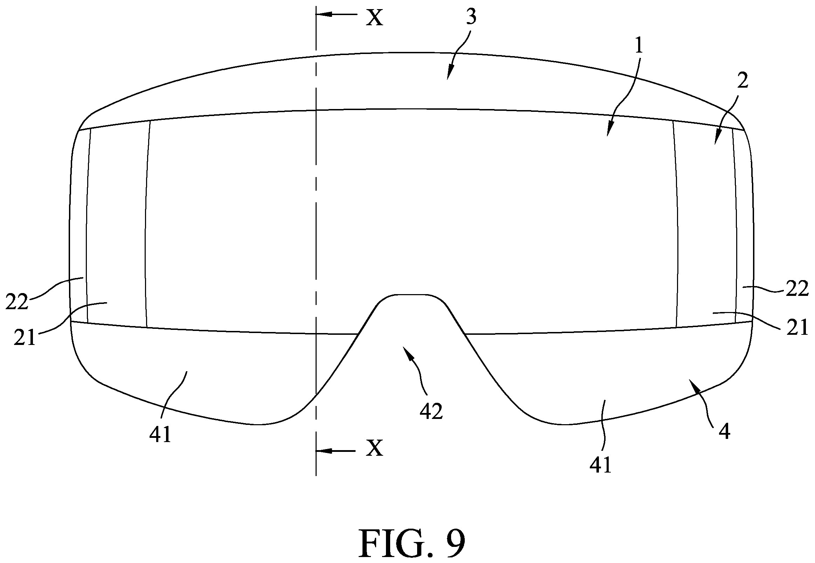

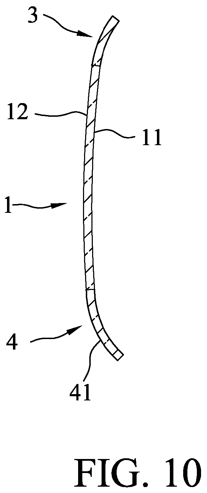

With reference to FIGS. 8, 9 and 10, in another embodiment, the goggle lens further includes an upper lens portion 3 and a lower lens portion 4. The upper lens portion 3 is integrally connected with and extends upwardly from upper edges of the central lens portion 1 and the lateral lens portions 2. The lower lens portion 4 is integrally connected with and extends downwardly from lower edges of the central lens portion 1 and the lateral lens portions 2. The lower lens portion 4 has two lower lens sections 41 which are spaced apart from each other by a nose recess 42.

The upper lens portion 3 and the lower lens portion 4 may have a variety of shapes to be coupled with the goggle frame 8 and to increase the field of views.

As illustrated, with the first horizontal radius of curvature of the central convex surface 12 being greater than the second horizontal radius of curvature of the first lateral lens section 21, the central lens portion 1 is closer and more fittingly attached to the wearer's face to reduce the visual deviation of the optical image, and the aesthetic appearance of the goggle lens is improved.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. A goggle lens adapted to be coupled with a goggle frame (8) for enclosing a wearer's head, comprising:
a central lens portion (1) including a central convex surface (12) distal to the wearer's face, said central convex surface (12) having a first horizontal radius of curvature which is defined by a curvature of a horizontal normal section between a transverse normal plane and any point on said central convex surface (12); and
two lateral lens portions (2) respectively extending from a left side and a right side of said central lens portion (1), each of said lateral lens portions (2) including a first lateral lens section (21) which has a first convex surface (212) distal to the wearer's face, said first convex surface (212) having a second horizontal radius of curvature which is defined by a curvature of a horizontal normal section between a transverse normal plane and any point on said first convex surface (212), **characterized in that**:
said second horizontal radius of curvature is smaller than said first horizontal radius of curvature.

2. The goggle lens of claim 1, wherein said first horizontal radius of curvature of said central convex surface (12) ranges from 109 mm to 234 mm.

3. The goggle lens of claim 2, wherein said central lens portion (1) has a refractive index ranging from 1.4 to 1.6.

4. The goggle lens of claim 3, wherein said second horizontal radius of curvature of said first convex surface (212) of each of said lateral lens portions (2) ranges from 41 mm to 73 mm.

5. The goggle lens of claim 3, wherein said central convex surface (12) of said central lens portion (1) has a first vertical radius of curvature which is defined by a curvature of a vertical normal section between a longitudinal normal plane and any point on said central convex surface (12), said first vertical radius of curvature being not smaller than said first horizontal radius of curvature.

6. The goggle lens of claim 5, wherein said first vertical radius of curvature of said central convex surface (12) ranges from 163 mm to 586 mm.

7. The goggle lens of claim 6, wherein said central lens portion (1) is made from a material including polycarbonate, said first horizontal radius of curvature of said central convex surface (12) ranging from 130 mm to 234 mm, said first vertical radius of curvature of said central convex surface (12) ranging from 195 mm to 586 mm, said second horizontal radius of curvature of said first convex surface (212) of each of said lateral lens portions (2) ranging from 49 mm to 73 mm.

8. The goggle lens of claim 3, wherein said central convex surface (12) of said central lens portion (1) has a first vertical radius of curvature which is defined by a curvature of a vertical normal section between a longitudinal normal plane and any point on said central convex surface (12), said first convex surface (212) of each of said lateral lens portions (2) having a second vertical radius of curvature which is defined by a curvature of a vertical normal section between a longitudinal normal plane and any point on said first convex surface (212), said first vertical radius of curvature being equal to said second vertical radius of curvature of said first convex surface (212) of each of said lateral lens portions (2).

9. The goggle lens of claim 3, wherein each of said lateral lens portions (2) further includes a second lateral lens section (22) which extends laterally from said first lateral lens section (21) and distal from said central lens portion (1), said second lateral lens section (22) having a second convex surface (222) distal to the wearer's face, said second convex surface (222) having a third horizontal radius of curvature which is defined by a curvature of a horizontal normal section between a transverse normal plane and any point on said second convex surface (222), said third horizontal radius of curvature being smaller than said first horizontal radius of curvature.

10. The goggle lens of claim 9, wherein said third horizontal radius of curvature of said second convex surface (222) of each of said lateral lens portions (2) ranges from 41 mm to 73 mm.

11. The goggle lens of claim 10, wherein said central convex surface (12) of said central lens portion (1) has a first vertical radius of curvature which is defined by a curvature of a vertical normal section between a longitudinal normal plane and any point on said central convex surface (12), said second convex surface (222) of each of said lateral lens portions (2) having a third vertical radius of curvature which is defined by a curvature of a vertical normal section between a longitudinal normal plane and any point on said second convex surface (222), said first vertical radius of curvature being equal to said third vertical radius of curvature of said second convex surface (222) of each of said lateral lens portions (2).

12. The goggle lens of claim 1, further comprising an upper lens portion (3) which is integrally connected with and extends upwardly from upper edges of said central lens portion (1) and said lateral lens portions (2), and a lower lens portion (4) which is integrally connected with and extends downwardly from lower edges of said central lens portion (1) and said lateral lens portions (2).
